(19) Europäisches Patentamt / European Patent Office / Office européen des brevets

(11) **EP 3 144 665 B1**

(12) **EUROPEAN PATENT SPECIFICATION**

(45) Date of publication and mention
of the grant of the patent:
**24.10.2018 Bulletin 2018/43**

(21) Application number: **15793449.8**

(22) Date of filing: **12.05.2015**

(51) Int Cl.:
*G01N 21/359* (2014.01)    *G01N 33/02* (2006.01)
*G01N 21/27* (2006.01)

(86) International application number:
**PCT/JP2015/002392**

(87) International publication number:
**WO 2015/174073 (19.11.2015 Gazette 2015/46)**

(54) **FOOD ANALYSIS DEVICE**

LEBENSMITTELANALYSEVORRICHTUNG

DISPOSITIF D'ANALYSE DE PRODUITS ALIMENTAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2014 JP 2014099193**

(43) Date of publication of application:
**22.03.2017 Bulletin 2017/12**

(73) Proprietor: **Panasonic Intellectual Property
Management Co., Ltd.
Osaka-shi, Osaka 570-6207 (JP)**

(72) Inventor: **OCHI, Kazuhiro
Osaka 540-6207 (JP)**

(74) Representative: **Grünecker Patent- und
Rechtsanwälte
PartG mbB
Leopoldstraße 4
80802 München (DE)**

(56) References cited:
**WO-A1-2005/111583    WO-A1-2009/038206
JP-A- 2003 270 139    JP-A- 2004 515 778
JP-A- 2004 530 875    JP-A- 2005 121 663
JP-A- 2011 257 218    US-A1- 2011 071 807
US-A1- 2012 260 743**

- **BECHMANN I E ET AL.: 'Rapid Assessment of
Quality Parameters for Frozen Cod Using Near
Infrared Spectroscopy' FOOD SCIENCE &
TECHNOLOGY vol. 31, 1998, ISSN 0023-6438
pages 648 - 652, XP055236531**
- **REIS M M ET AL.: 'Early on-line classification of
beef carcasses based on ultimate pH by near
infrared spectroscopy' MEAT SCIENCE vol. 96,
no. 2, February 2014, ISSN 0309-1740 pages 862
- 869, XP028789919**

EP 3 144 665 B1

## Description

### TECHNICAL FIELD

The present invention relates to a food analysis apparatus configured to analyze nutrition information of food.

### BACKGROUND ART

In the related art, there has been proposed a food analysis apparatus that calculates nutrition information such as calories and a constituent amount of food as an object for analysis, in a non-destructive manner. For example, there is an apparatus disclosed in PTL 1 as an example of the food analysis apparatus in the related art. Specifically, the apparatus disclosed in PTL 1 performs multiple regression analysis on the absorbance of various types of samples, which is obtained when the samples, of which calories are already known, are irradiated with far-red light. As a result, a regression equation representing a correlation between calories and the absorbance of the samples is created. The absorbance of food, which is obtained when the food as an object for analysis is irradiated with the far-red light under the same conditions, is substituted in the created regression equation such that calories of the food are calculated.
However, in the apparatus disclosed in PTL 1, in a case where a state of food as the object for analysis significantly differs from a state of the sample used in creation of the regression equation, correlations between calories and the absorbance of both the food as the object for analysis and the sample also significantly differ from each other in a similar manner. Therefore, although the absorbance of the food as the object for analysis is substituted in the regression equation, a problem arises in that it is not possible to calculate calories of the food with accuracy.
Note that it is possible to perform uniform analysis in any range of calories in theory; however, in practice, the analysis is susceptible to error and thus, an analysis result is obtained with low accuracy due to an influence of the error. In particular, in a case of calorie analysis, the analysis tends to be performed by focusing on a one-sided constitution, for example, as analysis of constituents of a low-calorie sample and constituents of a high-calorie sample. For example, since the high-calorie sample contains a lipid as a main constituent, a lipid-centered constitution is obtained. When a sample having a one-sided constitution is analyzed, light absorption of constituents of the low-calorie sample is increased and thus, variations due to error significantly influence an analysis result. Therefore, since a high-calorie sample contains a lipid as the main constituent, the analysis is performed with high accuracy using an equation specialized in estimation of a lipid without using errors included in analysis of other constituents in a regression equation. Conversely, since a vegetable contains a low amount of lipid, the analysis is performed with high accuracy by using an equation having a low influence of an error due to a wavelength of a lipid.
Note that such a problem is not limited to a case of calculating calories of food, and, for example, a similar problem arises in a case where a regression equation representing a correlation between the absorbance and nutrition information other than calories is created in advance in a case of calculating nutrition information other than calories of food, and the absorbance of the food as an object for analysis is substituted in the regression equation created in advance.

Citation List

Patent Literature

PTL 1: Japanese Patent Unexamined Publication No. 2008-122412
JP 2011 257218 A relates to a component analysis device for analyzing a component of an object. A memory stores a plurality of models for component analysis with each model correlated with an instruction. An operation device receives an instruction and a change device changes a state of an object in accordance with the instruction. A measurement device acquires data associated with reflected light or transmitted light from an object by irradiating the object with light. A processor reads a model corresponding to the instruction from the memory and uses the model to analyze a component of the object based upon the acquired data.

### SUMMARY OF THE INVENTION

The present invention is made in consideration of such problem in the food analysis apparatus in the related art. Specifically, one of the objects of the present invention it to provide a food analysis apparatus that can reduce an influence of an error on an analysis result, which occurs in a case where a state of food as an object for analysis significantly differs from a state of a sample used in creation of a regression equation, and that can measure nutrition information of the food as an object of the analysis with high accuracy.
The invention is defined by independent claim 1. As in the preamble of claim 1, a food analysis apparatus configured to analyze nutrition information of food comprises an irradiation unit configured to irradiate the food with light containing a wavelength in a near-infrared region; and a light receiving unit configured to receive, among the light emitted from the irradiation unit, light transmitted through the food or light reflected from the food. The food analysis apparatus further comprises a model storage unit configured to store a plurality of correlation models representing a correlation between nutrition information and absorbance of the plurality of samples, wherein the absorbance of each of the plurality of samples is calculated based on an amount of light reception that is measured by the light receiving unit when the irradiation unit irradiates, with the light, each of the plurality of samples whose nutrition information is already known, and is classified in association with feature information including at least one item of information of a state, a temperature, a light intensity, a weight, and a color of each of the plurality of samples. The food analysis apparatus further comprises an analysis unit configured to analyze the nutrition information of the food by selecting a correlation model from the plurality of correlation models stored in the model storage unit, and applying the absorbance of the food which is calculated based on the amount of light received by the light receiving unit when the food is irradiated with the light, to the selected correlation model.

By such configuration, it is possible to avoid an error in an analysis result, which occurs in a case where a state of the food as an object of the analysis significantly differs from a state of the sample used in creation of a regression equation, or the like, and it is possible to measure the nutrition information of the food as the analysis object, with high accuracy.

BRIEF DESCRIPTION OF DRAWINGS

FIG. 1 is a view schematically showing an entire structure of a food analysis apparatus of Exemplary Embodiment 1 of the present disclosure.
FIG. 2 is a view schematically showing a configuration of a light receiving unit of Exemplary Embodiment 1.
FIG. 3 is a block diagram showing a control configuration of an analysis unit of Exemplary Embodiment 1.
FIG. 4 is a diagram schematically showing a correlation between a sample and a correlation model in Exemplary Embodiment 1.
FIG. 5 is a graph schematically showing a correlation between distribution of a composition ratio of protein of a sample and a correlation model in Exemplary Embodiment 8 of the present disclosure, which is an example of the present invention.
FIG. 6 is a diagram schematically showing a correlation between light receiving elements of a light receiving sensor and a correlation model as an object to which an amount of light received by the light receiving elements is applied in Exemplary Embodiment 1.
FIG. 7 is a view schematically showing a configuration of a light receiving unit of a modification example of exemplary embodiments of the present disclosure.
FIG. 8 is a view schematically showing an entire structure of the food analysis apparatus of a modification example of exemplary embodiments of the present disclosure.

DESCRIPTION OF EMBODIMENTS

(Exemplary Embodiment 1)

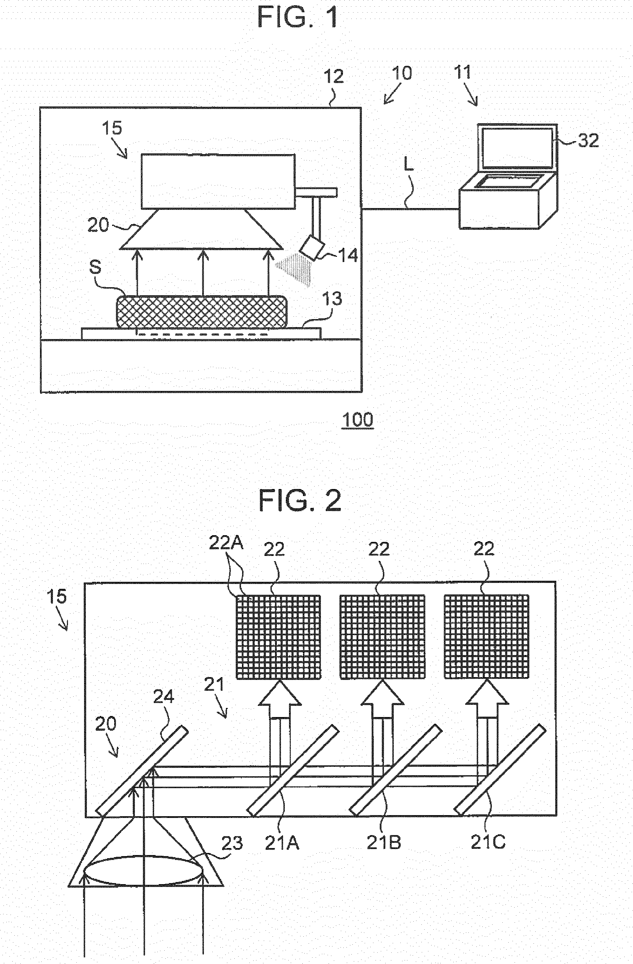

Hereinafter, Exemplary Embodiment 1 of a food analysis apparatus will be described with reference to the figures.
As shown in FIG. 1, food analysis apparatus 100 includes measurement unit 10 and analysis unit 11. Measurement unit 10 and analysis unit 11 are electrically connected via connection cable L.
Measurement unit 10 includes housing 12 that can block light from being emitted from the inside of the housing when a door is closed. Table 13, light emitting unit 14, and light receiving unit 15 are accommodated in housing 12. Target object S which is food as an object for analysis is mounted on table 13. Light emitting unit 14 functions as an irradiation unit which is configured to irradiate entire object S with light containing a wavelength of at least 700 nm to 2500 nm in a near-infrared region. In this case, light emitting unit 14 which is the irradiation unit may be configured to partially irradiate object S with light containing a wavelength in the near-infrared region. Note that, for example, a halogen lamp, a light emitting diode, a laser, or the like is used as light emitting unit 14.
In addition, instead of light emitting unit 14, for example, a slit, through which the inside and the outside of housing 12 communicate with each other, may be formed, and the slit may function as an irradiation unit which irradiates the object S with sunlight which is guided from the outside of housing 12. In this case, a filter, which selectively transmits light having a wavelength in the near-infrared region, may be provided in the slit of housing 12.
In addition, such a filter may not be provided and the slit may be configured to directly guide, to object S, sunlight containing a wavelength in a region other than the near-infrared region.

As shown in FIG. 2, light receiving unit 15 includes light collecting unit 20, spectroscopic unit 21, and light receiving sensor 22. Light collecting unit 20 includes collecting lens 23 and reflective unit 24. Collecting lens 23 collects light reflected from object S. Reflective portion 24 guides light collected by collecting lens 23 to spectroscopic unit 21.

Spectroscopic portion 21 includes first spectroscopic unit 21A, second spectroscopic unit 21B, and third spectroscopic unit 21C. As spectroscopic units 21A to 21C, a grating spectroscope, which reflects only light having a specific wavelength and transmits light having another wavelength, is used. Note that an interference filter-type spectroscope may be used as spectroscopic units 21A to 21C. In addition, the light beams, which are selectively reflected from spectroscopic units 21A to 21C, have different specific wavelengths from each other. The specific wavelengths are determined through an experiment or the like, based on the absorbance of a plurality of samples of which nutrition information such as a constituent amount or the like of food is already known. Specifically, the specific wavelengths are determined to be a wavelength obtained by reflecting a constituent amount of a specific constituent in a sample, which is selected from a relationship between the constituent amount and the absorbance of the specific constituent in a plurality of samples.

In the exemplary embodiment, first spectroscopic unit 21A selectively reflects light having a wavelength of about 910 nm as a wavelength obtained by reflecting a constituent amount of protein in a sample. In addition, second spectroscopic unit 21B selectively reflects light having a wavelength of about 930 nm as a wavelength obtained by reflecting a constituent amount of a lipid in a sample. In addition, third spectroscopic unit 21C selectively reflects light having a wavelength of about 980 nm as a wavelength obtained by reflecting a constituent amount of a carbohydrate in a sample.

Light receiving sensor 22 is a so-called image sensor, and a plurality of light receiving elements 22A are arranged in a grid shape on a light receiving surface of the sensor. As a material of light receiving elements 22A, silicon, indium, gallium, arsenic, and the like, which are sensitive to light having a wide wavelength range in the near-infrared region, are used. Note that, as the material of light receiving element 22A, a material, which is selectively sensitive to light having a specific wavelength obtained by reflecting constituent amounts of constituents such as protein described above in the sample, may be used.

Next, a configuration of analysis unit 11 will be described.

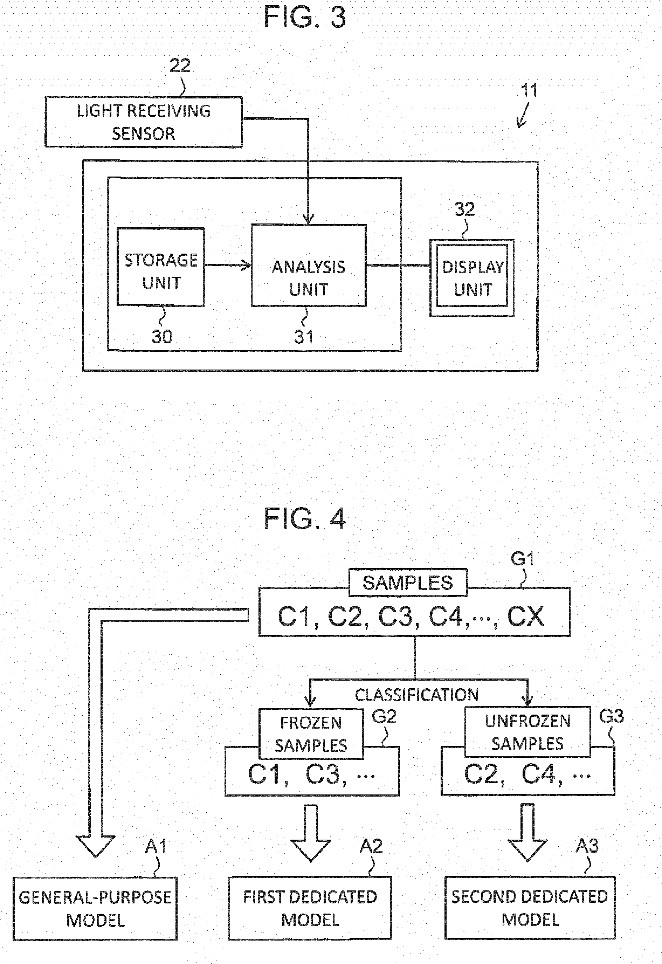

As shown in FIG. 3, analysis unit 11 includes storage unit 30 which is a model storage unit, analysis unit 31, and display unit 32.

Storage unit 30 which is the model storage unit retains an estimation model (hereinafter, referred to as a correlation model) representing a correlation between nutrition information (hereinafter, simply referred to as a constituent amount) of a constituent amount or the like of a sample and the absorbance of the sample that is calculated based on an amount of light reception measured by light receiving sensor 22 of light receiving unit 15, when the plurality of samples are irradiated with light from light emitting unit 14 which is the irradiation unit. In this case, the constituent amounts of the plurality of samples are calculated in advance by another analysis method in the related art with high accuracy.

Thus, a statistical method such as multiple linear regression analysis and partial least squares (PLS) is applied to the constituent amount and the absorbance of the sample, and thereby a correlation model representing the correlation is calculated in advance. For example, as in the following Equation (1), the correlation model is represented by a general polynomial equation using the absorbance of a sample with respect to light having a plurality of wavelengths.

$$Y = K0 + K1 \cdot f1\,(\lambda 1) + K2 \cdot f2\,(\lambda 2) + \cdots + Kn \cdot fn\,(\lambda n)\ \cdots\ (1)$$

[Y: correlation model, fm $(\lambda m)$(m = 1, 2, $\cdots$ n): absorbance of sample with respect to light having wavelength $\lambda m$, Km (m = 0, 1, 2, $\cdots$ n): proportional constant]

Note that, as shown in FIG. 4, in the exemplary embodiment, with respect to group G1 of all of X samples C1, C2, C3, C4, $\cdots$, CX, of which constituent amounts are calculated in advance, general-purpose model A1 which is a first correlation model is calculated in advance as the correlation model representing the correlation between the absorbance of the sample and the constituent amount of the sample. In addition, of the X samples C1, C2, C3, C4, $\cdots$, CX, with respect to group G2 of the samples C1, C3, $\cdots$ classified to be in a frozen state, first dedicated model A2 which is a second correlation model is calculated as the correlation model representing the correlation between the absorbance of the sample and the constituent amount of the sample. In addition, of the X samples C1, C2, C3, C4, $\cdots$, CX, with respect to group G3 of the samples C2, C4, $\cdots$ classified to be in an unfrozen state, second dedicated model A3 which is a second correlation model is calculated as the correlation model representing the correlation between the absorbance of the sample and the constituent amount of the sample.

In other words, in the present exemplary embodiment, when the plurality of samples, of which the constituent amounts are already known, are irradiated with light from light emitting unit 14 which is the irradiation unit, the absorbance of the samples, which is calculated based on an amount of light reception measured by light receiving sensor 22 of light receiving unit 15, is classified in association with feature information of the sample on whether the sample is in the frozen state. Then, first dedicated model A2 and second dedicated model A3, which are the second correlation model, are calculated, respectively, in advance, as the correlation model representing the correlation between the classified absorbance of each of group G2 and group G3 of the samples and the constituent amounts of the samples.

The plurality of correlation models are retained in storage unit 30 which is the model storage unit.

Analysis unit 31 applies, to the correlation model retained in storage unit 30, the absorbance of object S calculated based on the amount of light reception measured by light receiving sensor 22 when object S is irradiated with light from light emitting unit 14, thereby analyzing a constituent amount of object S.

In the present exemplary embodiment, analysis unit 31 configured to uniformly apply the absorbance for each analysis site of object S calculated based on an amount of light received by all of light receiving elements 22A of light receiving sensor 22 of light receiving unit 15, to a correlation model (for example, the first correlation model) selected from the plurality of correlation model retained in storage unit 30.

In addition, analysis unit 31 outputs and displays an analysis result of the constituent amounts of object S to display unit 32. Specifically, when object S is in the frozen state, analysis unit 31 selects and reads first dedicated model A2, which is the second correlation model, from storage unit 30. Then, the analysis unit applies the absorbance of object S to first dedicated model A2, and thereby analyzing a constituent amount of object S. By comparison, when object S is in the unfrozen state, analysis unit 31 selects and reads second dedicated model A3, which is the second correlation model, from storage unit 30. Then, the analysis unit applies the absorbance of object S to second dedicated model A3, and thereby analyzing a constituent amount of object S.

Next, an operation of food analysis apparatus 100 of the present exemplary embodiment will be described.

General-purpose model A1, which is the first correlation model, is a model having high general-purpose properties in that the general-purpose model represents the correlation between the absorbance of the samples and the constituent amounts of the samples, with respect to all of the samples of which the constituent amounts are calculated in advance, and includes many samples as objects. However, with respect to the frozen samples and the unfrozen samples of the samples used when general-purpose model A1 is created, the correlations between the absorbance and the constituent amounts of the samples tend to significantly differ from each other. In other words, the correlations between the absorbance and the constituent amounts of the samples tend to significantly differ from each other, depending on the state of the sample.

Therefore, when the absorbance of object S in the frozen state is applied to general-purpose model A1 and a constituent amount of object S is calculated, the analysis result is likely to contain an error because the samples in the unfrozen state are included in the samples used when general-purpose model A1 is created. Similarly, when the absorbance of object S in the unfrozen state is applied to general-purpose model A1 and a constituent amount of object S is calculated, the analysis result is likely to contain an error because the samples in the frozen state are included in the samples used when general-purpose model A1 is created.

In this respect, in the exemplary embodiment, when object S in the frozen state is the analysis object, the constituent amount of object S is analyzed by using first dedicated model A2 created based on the samples in the frozen state. By comparison, when object S in the unfrozen state is the analysis object, the constituent amount of object S is analyzed by using second dedicated model A3 created based on the samples in the unfrozen state. Therefore, an occurrence of error in the analysis result is reduced, compared to a case where general-purpose model A1 is uniformly applied so as to analyze the constituent amount of object S, regardless of whether or not object S is in the frozen state.

Food analysis apparatus 100 of the exemplary embodiment achieves the following effect.

(1) A correlation model corresponding to the state of object S is selected from a plurality of correlation models stored in storage unit 30. Then, a constituent amount of object S is analyzed using the selected correlation model. Therefore, it is possible to measure the constituent amount of object S with high accuracy, compared to a case where a correlation model is uniformly applied so as to analyze the constituent amount of object S, regardless of a state of object S.

(Exemplary Embodiment 2)

Next, Exemplary Embodiment 2 of food analysis apparatus 100 will be described. Note that Exemplary Embodiment 2 differs from Exemplary Embodiment 1 in that a correlation model used in analysis of a constituent amount of object S is switched depending on a temperature of object S. Therefore, in the following description, a configuration different from that in Exemplary Embodiment 1 will be mainly described, and repeated description of the same or similar configuration as that in Exemplary Embodiment 1 will be omitted.

In the exemplary embodiment, with respect to group G1 of all of the samples of which the constituent amounts are calculated in advance, general-purpose model A1, which is the first correlation model, is calculated in advance as the correlation model representing the correlation between the absorbance of the sample and the constituent amount of the sample.

In addition, of the samples, with respect to a group of the classified samples which have a temperature of 0°C or higher, a first dedicated model, which is the second correlation model, is calculated in advance as the correlation model representing the correlation between the absorbance of the sample and the constituent amount of the sample.

In addition, of the samples, with respect to a group of the classified samples which have a temperature lower than of 0°C, a second dedicated model, which is the second correlation model, is calculated in advance as the correlation model representing the correlation between the absorbance of the sample and the constituent amount of the sample.

In other words, in the present exemplary embodiment, when the plurality of samples, of which the constituent amounts are already known, are irradiated with light from light emitting unit 14 which is the irradiation unit, the absorbance of the samples, which is calculated based on an amount of light reception measured by light receiving sensor 22 of light receiving unit 15, is classified in association with feature information of the sample on whether the sample has the temperature of 0°C or higher. In this manner, the first dedicated model and the second dedicated model, which are the second correlation model, are calculated, respectively, in advance, as the correlation model representing the correlation between the classified absorbance of the respective groups of the samples and the constituent amounts of the samples. Note that, for example, the temperature of the sample is measured by using a radiation thermometer, a contact thermometer, or the like.

Here, with respect to the samples having the temperature of 0°C or higher and the samples having the temperature of lower than 0°C of the samples used when general-purpose model A1 is created, the correlations between the absorbance and the constituent amounts of the samples tend to significantly differ from each other. In other words, the correlations between the absorbance and the constituent amounts of the samples tend to significantly differ from each other, depending on the temperature of the sample.

Therefore, when the absorbance of object S having the temperature of 0°C or higher is applied to general-purpose model A1 and a constituent amount of object S is calculated, the analysis result is likely to contain an error because the samples having the temperature of lower than 0°C are included in the samples used when general-purpose model A1 is created. Similarly, when the absorbance of object S having the temperature of lower than 0°C is applied to general-purpose model A1 and a constituent amount of object S is calculated, the analysis result is likely to contain an error because the samples having the temperature of 0°C or higher are included in the samples used when general-purpose model A1 is created.

In this respect, in the exemplary embodiment, when object S having the temperature of 0°C or higher is the analysis object, the constituent amount of object S is analyzed by using the first dedicated model created based on the samples having the temperature of lower than 0°C. By comparison, when object S having the temperature of lower than 0°C is the analysis object, the constituent amount of object S is analyzed by using the second dedicated model created based on the samples having the temperature of lower than 0°C. Therefore, an occurrence of error in the analysis result is reduced, compared to a case where general-purpose model A1 is uniformly applied so as to analyze the constituent amount of object S, regardless of whether or not object S has the temperature of 0°C or higher.

Food analysis apparatus 100 of Exemplary Embodiment 2 achieves the same effect (1) as in Exemplary Embodiment 1 described above.

(Exemplary Embodiment 3)

Next, Exemplary Embodiment 3 of food analysis apparatus 100 will be described. Note that Exemplary Embodiment 3 differs from Exemplary Embodiment 1 in that a correlation model used in analysis of a constituent amount of object S is switched depending on an amount of water in object S. Therefore, in the following description, a configuration different from that in Exemplary Embodiment 1 will be mainly described, and repeated description of the same or similar configuration as that in Exemplary Embodiment 1 will be omitted.

In the exemplary embodiment, with respect to group G1 of all of the samples of which the constituent amounts are calculated in advance, general-purpose model A1, which is the first correlation model, is calculated in advance as the correlation model representing the correlation between the absorbance of the sample and the constituent amount of the sample.

In addition, of the samples, with respect to a group of the classified samples which are samples containing an amount of water greater than an amount of water that is normally (under a condition in which the object is normally disposed, for example, disposed in the atmosphere at room temperature in a case of a vegetable, hereinafter, all the same) contained in object S, the first dedicated model, which is the first correlation model, is calculated in advance as the correlation model representing the correlation between the absorbance of the sample and the constituent amount of the sample.

In addition, of the samples, with respect to a group of the classified samples which are samples containing a normal amount of water, the second dedicated model, which is the second correlation model, is calculated in advance as the correlation model representing the correlation between the absorbance of the sample and the constituent amount of the sample.

In addition, of the samples, with respect to a group of the classified samples which are samples containing an amount of water smaller than the normal amount, a third dedicated model, which is the second correlation model, is calculated in advance as the correlation model representing the correlation between the absorbance of the sample and the constituent amount of the sample.

In other words, in the exemplary embodiment, when the plurality of samples, of which the constituent amounts are already known, are irradiated with light from light emitting unit 14 which is the irradiation unit, the absorbance of the samples, which is calculated based on an amount of light reception measured by light receiving sensor 22 of light receiving unit 15, is classified in association with feature information of the sample on whether the amount of water in the sample is equal to the amount of water normally contained in the sample. In this manner, the first dedicated model, the second dedicated model, and the third dedicated model are calculated, respectively, in advance, as the correlation model representing the correlation between the classified absorbance of the respective groups of the samples and the constituent amounts of the samples. Note that, for example, the amount of water in the sample is measured based on an amount of light absorption of a wavelength in the near-infrared region in the sample, electroconductivity of the sample, or the like.

Here, water has an absorbing spectrum in a wide wavelength range with 960 nm and 1500 nm as the center. Therefore, the correlations between the absorbance and the constituent amounts of the samples tend to significantly differ from each other, with respect to the samples having the normal amount of water and the samples having abnormal amounts of water of the samples used when general-purpose model A1 is created. In other words, the correlations between the absorbance and the constituent amounts of the samples tend to significantly differ from each other, depending on the amount of water.

In this respect, in the exemplary embodiment, when object S containing the amount of water greater than the normal amount is the analysis object, the constituent amount of object S is analyzed by using the first dedicated model created based on the samples containing the amount of water greater than the normal amount.

In addition, when object S containing the normal amount of water is the analysis object, the constituent amount of object S is analyzed by using the second dedicated model created based on the samples containing the normal amount of water.

In addition, when object S containing the amount of water smaller than the normal amount is the analysis object, the constituent amount of object S is analyzed by using the third dedicated model created based on the samples containing the amount of water smaller than the normal amount.

Therefore, an occurrence of error in the analysis result is reduced, compared to a case where a general-purpose model is uniformly applied so as to analyze the constituent amount of object S, regardless of whether or not object S has the normal amount of water.

The food analysis apparatus of Exemplary Embodiment 3 achieves the same effect (1) as in Exemplary Embodiment 1 described above.

(Exemplary Embodiment 4)

Next, Exemplary Embodiment 4 of food analysis apparatus 100 will be described. Note that Exemplary Embodiment 4 differs from Exemplary Embodiment 1 in that a correlation model used in analysis of a constituent amount of object S is switched depending on a light intensity of object S. Therefore, in the following description, a configuration different from that in Exemplary Embodiment 1 will be mainly described, and repeated description of the same or similar configuration as that in Exemplary Embodiment 1 will be omitted.

In the exemplary embodiment, with respect to group G1 of all of the samples of which the constituent amounts are calculated in advance, general-purpose model A1 is calculated in advance as the correlation model representing the correlation between the absorbance of the sample and the constituent amount of the sample.

In addition, of the samples, with respect to a group of the classified samples which are samples having a light intensity higher than a predetermined light intensity, the first dedicated model is calculated in advance as the correlation model representing the correlation between the absorbance of the sample and the constituent amount of the sample.

In addition, of the samples, with respect to a group of the classified samples which are samples having a light intensity lower than the predetermined light intensity, the second dedicated model is calculated in advance as the correlation model representing the correlation between the absorbance of the sample and the constituent amount of the sample.

In other words, in the exemplary embodiment, when the plurality of samples, of which the constituent amounts are already known, are irradiated with light from light emitting unit 14 which is the irradiation unit, the absorbance of the samples, which is calculated based on an amount of light reception measured by light receiving sensor 22, is classified in association with feature information of the sample on whether the sample has a high light intensity. In this manner, the first dedicated model and the second dedicated model are calculated, respectively, in advance, as the correlation model representing the correlation between the classified absorbance of the respective groups of the samples and the constituent amounts of the samples. Note that, for example, the light intensity of the sample is measured using the absorbance of the sample with respect to light having a wavelength in the near-infrared region or in a visible light region. In addition, the light intensity of the sample may be measured using the absorbance of the sample with respect to light in the entire wavelength range. In addition, the light intensity of the sample may be measured based on a tone of the sample, which is calculated by performing an image analysis process on an image of the captured sample.

Here, the light intensity incident to light receiving sensor 22 varies depending on density, a size, a color, or the like, of the sample. For example, in a case of a white sample and in a case of dense crystalline state, the sample has high reflection ratio, and very high light intensity is incident to light receiving sensor 22. Therefore, with respect to the samples having substantially the same level of the light intensity as the predetermined light intensity set in advance, as the normal light intensity of object S, and the samples having the abnormal light intensity, of the samples used when the general-purpose model A1 is created, the correlations between the absorbance and the constituent amounts of the samples tend to significantly differ from each other. In other words, the correlations between the absorbance and the constituent amounts of the samples tend to significantly differ from each other, depending on the light intensity of the sample.

In this respect, in the exemplary embodiment, when object S having the light intensity higher than the predetermined light intensity is the analysis object, the constituent amount of object S is analyzed by using the first dedicated model created based on the samples having the light intensity higher than the predetermined light intensity.

In addition, when object S having the light intensity lower than the predetermined light intensity is the analysis object, the constituent amount of object S is analyzed by using the second dedicated model created based on the samples having the light intensity lower than the predetermined light intensity.

Therefore, an occurrence of error in the analysis result is reduced, compared to a case where a general-purpose model is uniformly applied so as to analyze the constituent amount of object S, regardless of whether or not object S has the normal light intensity.

The food analysis apparatus of Exemplary Embodiment 4 achieves the same effect (1) as in Exemplary Embodiment 1 described above.


(Exemplary Embodiment 5)

Next, Exemplary Embodiment 5 of food analysis apparatus 100 will be described. Note that Exemplary Embodiment 5 differs from Exemplary Embodiment 1 in that a correlation model used in analysis of a constituent amount of object S is switched depending on a size of object S. Therefore, in the following description, a configuration different from that in Exemplary Embodiment 1 will be mainly described, and repeated description of the same or similar configuration as that in Exemplary Embodiment 1 will be omitted.

In the exemplary embodiment, with respect to group G1 of all of the samples of which the constituent amounts are calculated in advance, general-purpose model A1 is calculated in advance as the correlation model representing the correlation between the absorbance of the sample and the constituent amount of the sample.

In addition, of the samples, with respect to a group of the classified samples which are samples having a size larger than a normal size (size set in advance as the normal size with respect to object S), the first dedicated model is calculated in advance as the correlation model representing the correlation between the absorbance of the sample and the constituent amount of the sample.

In addition, of the samples, with respect to a group of the classified samples which are samples having substantially the same size that object S normally has, the second dedicated model is calculated in advance as the correlation model representing the correlation between the absorbance of the sample and the constituent amount of the sample.

In addition, of the samples, with respect to a group of the samples classified to have a size smaller than the size of normal object S, the third dedicated model is calculated in advance as the correlation model representing the correlation between the absorbance of the sample and the constituent amount of the sample.

In other words, in the exemplary embodiment, when the plurality of samples, of which the constituent amounts are already known, are irradiated with light from light emitting unit 14, the absorbance of the samples, which is calculated based on an amount of light received by light receiving sensor 22, is classified in association with feature information of the sample on whether the sample has the normal size. In this manner, the first dedicated model, the second dedicated model, and the third dedicated model are calculated, respectively, in advance, as the correlation models representing the correlations between the classified absorbance of the respective groups of the samples and the constituent amounts of the samples.

Note that the size of the sample is calculated by measuring a height, a width, and the like of the sample, for example, using a distance sensor provided above the sample. In addition, the size of the sample may be calculated by measuring the height of the sample, using a pressure-sensitive sensor provided above the sample so as to be lifted and lowered. In addition, the size of the sample may be calculated by measuring an area of a bottom surface of the sample, using a pressure-sensitive sensor provided on the top surface of table 13. In addition, the size of the sample may be calculated based on a profile of the sample, which is extracted by performing an image analysis process with respect to an image of the captured sample. In addition, the size of the sample may be calculated by measuring an area of a light receiving surface of light receiving sensor 22, in which light having a wavelength in the near-infrared region is detected as the reflective light from the sample.

Here, in a case where the size of the sample is not the predetermined normal side, a ratio of the constituent amount of the sample to a total weight of the sample varies. Therefore, an influence of an error of a value of the absorbance of the sample which defines the correlation model, on a value of the constituent amount also varies in size. As a result, with respect to the samples having the normal size and the samples having abnormal sizes of the samples defining the genera-purpose model, the correlations between the absorbance and the constituent amounts of the samples tend to significantly differ from each other. In other words, the correlations between the absorbance and the constituent amounts of the samples tend to significantly differ from each other, depending on the size of the sample.

In this respect, in the exemplary embodiment, when object S having the size larger than the predetermined normal size is the analysis object, the constituent amount of object S is analyzed by using the first dedicated model created based on the samples having the size larger than the predetermined normal size. In addition, when object S having substantially the same size as the predetermined normal size is the analysis object, the constituent amount of object S is analyzed by using the second dedicated model created based on the samples having the predetermined normal size. In addition, when object S having the size lower than the predetermined normal size is the analysis object, the constituent amount of object S is analyzed by using the third dedicated model defined based on the samples having the size smaller than the predetermined normal size. Therefore, an occurrence of error in the analysis result is reduced, compared to a case where a general-purpose model is uniformly applied so as to analyze the constituent amount of object S, regardless of whether or not the size of object S is the predetermined normal size.

The food analysis apparatus of Exemplary Embodiment 5 achieves the same effect (1) as in Exemplary Embodiment 1 described above.

(Exemplary Embodiment 6)

Next, Exemplary Embodiment 6 of food analysis apparatus 100 will be described. Note that Exemplary Embodiment 6 differs from Exemplary Embodiment 1 in that a correlation model used in analysis of a constituent amount of an object is switched depending on a weight of object S. Therefore, in the following description, a configuration different from that in Exemplary Embodiment 1 will be mainly described, and repeated description of the same or similar configuration as that in Exemplary Embodiment 1 will be omitted.

In the exemplary embodiment, with respect to group G1 of all of the samples of which the constituent amounts are calculated in advance, general-purpose model A1 is calculated in advance as the correlation model representing the correlation between the absorbance of the sample and the constituent amount of the sample.

In addition, of the samples, with respect to a group of the classified samples having a weight heavier than a normal weight (for example, under conditions in which object S is normally disposed, the weight of object S having the predetermined size is set in advance as the weight that object S normally has), the first dedicated model is calculated in advance as the correlation model representing the correlation between the absorbance of the sample and the constituent amount of the sample.

In addition, of the samples, with respect to a group of the classified samples having substantially the same weight that object S normally has, the second dedicated model is calculated in advance as the correlation model representing the correlation between the absorbance of the sample and the constituent amount of the sample.

In addition, of the samples, with respect to a group of the classified samples having a weight lighter than a normal weight, the third dedicated model is calculated in advance as the correlation model representing the correlation between the absorbance of the sample and the constituent amount of the sample.

In other words, in the exemplary embodiment, when the plurality of samples, of which the constituent amounts are already known, are irradiated with light from light emitting unit 14 which is the irradiation unit, the absorbance of the samples, which is calculated based on an amount of light reception measured by light receiving sensor 22, is classified in association with feature information of the sample on whether the sample has the predetermined normal weight. In this manner, the first dedicated model, the second dedicated model, and the third dedicated model are calculated, respectively, in advance, as the correlation models representing the correlations between the classified absorbance of the respective groups of the samples and the constituent amounts of the samples. Note that the weight of the sample is measured, for example, using a strain gauge and a piezoelectric element provided on the table 13.

Here, in a case where the sample does not have the normal weight, such as in a case where the sample is a trace object in a unit of milligram or, in a case where the sample is for an industrial use in a unit of kilogram, a ratio of the constituent amount of the sample to a total weight of the sample varies. Therefore, an influence of an error of a value of the absorbance of the sample which defines the correlation model, on a value of the constituent amount also varies in size. As a result, with respect to the samples having the normal weight and the samples having abnormal weight of the samples defining the genera-purpose model, the correlations between the absorbance and the constituent amounts of the samples tend to significantly differ from each other. In other words, the correlations between the absorbance and the constituent amounts of the samples tend to significantly differ from each other, depending on the weight of the sample.

In this respect, in the exemplary embodiment, when object S having the weight heavier than a weight set in advance as the normal weight of object S is the analysis object, the constituent amount of object S is analyzed by using the first dedicated model created based on the samples having the size heavier than the predetermined normal weight. In addition, when object S having substantially the same weight as the predetermined normal size is the analysis object, the constituent amount of object S is analyzed by using the second dedicated model created based on the samples having the predetermined normal size. In addition, when object S having the weight lighter than the predetermined normal weight is the analysis object, the constituent amount of object S is analyzed by using the third dedicated model created based on the samples having the weight lighter than the predetermined normal weight.

Therefore, an occurrence of error in the analysis result is reduced, compared to a case where a general-purpose model is uniformly applied so as to analyze the constituent amount of object S, regardless of whether or not object S is has the normal weight.

The food analysis apparatus of Exemplary Embodiment 6 achieves the same effect (1) as in Exemplary Embodiment 1 described above.

(Exemplary Embodiment 7)

Next, Exemplary Embodiment 7 of food analysis apparatus 100 will be described. Note that Exemplary Embodiment 7 differs from Exemplary Embodiment 1 in that a correlation model used in analysis of a constituent amount of object S is switched depending on an amount of water in object S. Therefore, in the following description, a configuration different from that in Exemplary Embodiment 1 will be mainly described, and repeated description of the same or similar configuration as that in Exemplary Embodiment 1 will be omitted.

In the exemplary embodiment, with respect to group G1 of all of the samples of which the constituent amounts are calculated in advance, general-purpose model A1 is calculated in advance as the correlation model representing the correlation between the absorbance of the sample and the constituent amount of the sample.

In addition, of the samples, for example, with respect to a group of black samples, the first dedicated model is calculated in advance as the correlation model representing the correlation between the absorbance of the sample and the constituent amount of the sample.

In addition, of the samples, for example, with respect to a group of green samples, the second dedicated model is calculated in advance as the correlation model representing the correlation between the absorbance of the sample and the constituent amount of the sample.

In other words, in the exemplary embodiment, when the plurality of samples, of which the constituent amounts are already known, are irradiated with light from light emitting unit 14 which is the irradiation unit, the absorbance of the samples, which is calculated based on an amount of light reception measured by light receiving sensor 22, is classified in association with feature information of the sample on the color of the sample. In this manner, the first dedicated model and the second dedicated model are calculated, respectively, in advance, as the correlation models representing the correlations between the classified absorbance of the respective groups of the samples and the constituent amounts of the samples. Note that a color image sensor is employed as light receiving sensor 22, and the color of the sample is measured, for example, based on a detection result from light receiving sensor 22.

Here, in a case where the color of the sample is black, the absorbance of the sample tends to increase in the entire wavelength range including the visible light region and the near-infrared region. Therefore, the correlations between the absorbance and the constituent amounts of the samples tend to significantly differ from each other depending on whether the color of the sample is black.

In addition, in a case where the color of the sample is green, the type of sample is highly likely to a type of vegetable that does not include much protein. Therefore, the correlations between the absorbance and the constituent amounts of the samples tend to significantly differ from each other depending on whether the color of the sample is green.

In this respect, in the exemplary embodiment, when black object S is the analysis object, the constituent amount of object S is analyzed by using the first dedicated model created based on the black samples. In addition, when green object S is the analysis object, the constituent amount of object S is analyzed by using the second dedicated model created based on the green samples. Therefore, an occurrence of error in the analysis result is reduced, compared to a case where a general-purpose model is uniformly applied so as to analyze the constituent amount of object S, regardless of the color of object S.

The food analysis apparatus of Exemplary Embodiment 7 achieves the same effect (1) as in Exemplary Embodiment 1 described above.

(Exemplary Embodiment 8)

Next, Exemplary Embodiment 8 of food analysis apparatus 100 will be described with reference to the figures. Note that Exemplary Embodiment 8 differs from Exemplary Embodiment 1 in that it is determined whether or not an object to which the absorbance of object S is applied is switched from a general-purpose model to a dedicated model, based on an analysis result of a constituent amount of object S obtained when the absorbance of object S is applied to the general-purpose model. Therefore, in the following description, a configuration different from that in Exemplary Embodiment 1 will be mainly described, and repeated description of the same or similar configuration as that in Exemplary Embodiment 1 will be omitted.

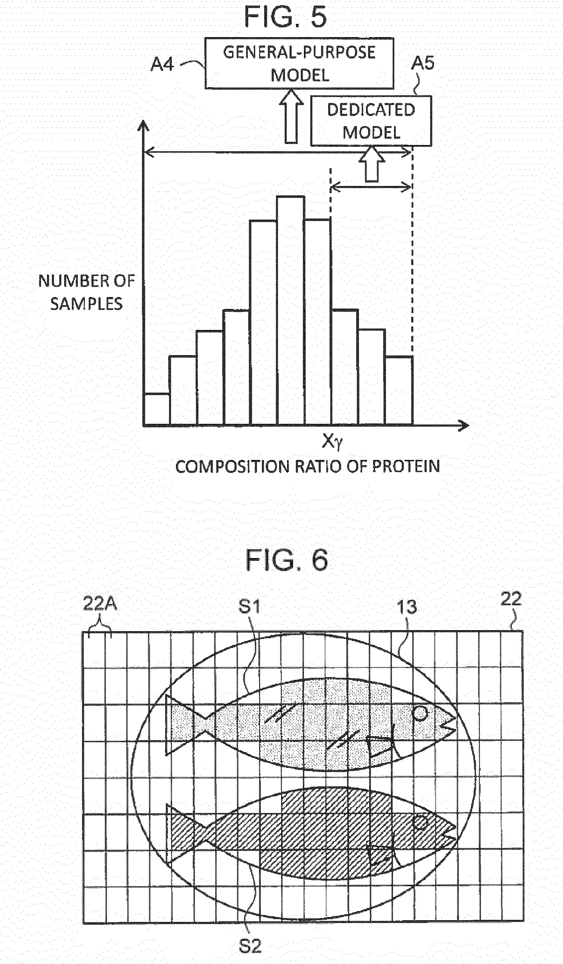

FIG. 5 shows distribution of a composition ratio of protein in a group of all of the samples. The distribution forms a shape close to normal distribution. In addition, in the exemplary embodiment, with respect to a group of all of the samples, general-purpose model A4, which is the first correlation model, is calculated in advance as the correlation model representing the correlation between the absorbance of the sample and the constituent amount of the sample.

In addition, of all of the samples, with respect to a group of classified samples of which a composition ratio of protein is equal to or higher than threshold value $X_\gamma$, dedicated model A5, which is the second correlation model, is calculated in advance as the correlation model representing the correlation between the absorbance of the sample and the constituent amount of the protein of the sample.

Then, analysis unit 31, first, applies the absorbance of object S to general-purpose model A4, thereby, analyzing the constituent amount of the protein of object S. Subsequently, it is determined whether or not the composition ratio of the protein of object S is equal to or higher than threshold value Xr. As a result, when the composition ratio of the protein of object S is equal to or higher than threshold value Xr, the absorbance of object S is applied to dedicated model A5, and thereby the constituent amount of the protein of object S is reanalyzed. By contrast, when the composition ratio of the protein of object S is lower than threshold value Xr, the constituent amount of the protein of object S is not reanalyzed.

Here, a sample having very low composition ratio of the protein is included in the group of all of the samples. Then, in such a sample, an error of a value of the absorbance of the sample, which is input to the correlation model, is likely to have relatively significant influence on an analysis value of the constituent amount of the protein. Therefore, in the exemplary embodiment, when the composition ratio of the protein of object S is equal to or higher than threshold value Xr, a sample, of which the composition ratio of the protein is inclined to be small, is ruled out. Then, dedicated model A5, which is the second correlation model, is created, and the constituent amount of the protein of object S is analyzed using dedicated model A5. As a result, an occurrence of error in the analysis result is reduced, compared to a case where general-purpose model A4 is uniformly applied so as to analyze the constituent amount of the protein of object S, regardless of the composition ratio of the protein of object S.

Food analysis apparatus 100 of Exemplary Embodiment 8 achieves the following effects, in addition to the same effect (1) as in Exemplary Embodiment 1 described above.

(2) The correlation model is more appropriately reselected as necessary, based on the analysis result of the constituent amount of the protein of object S, and then the constituent amount of the protein of object S is analyzed. Therefore, it is possible to measure the constituent amount of the protein of object S with higher accuracy.

(3) When the analysis result of the composition ratio of the protein of object S is equal to or higher than threshold value Xr, the constituent amount of the protein of object S is analyzed using dedicated model A5 corresponding to a sample of which the composition ratio of the protein is equal to or higher than threshold value Xr, of the plurality of samples. In other words, when the absorbance of object S is applied to general-purpose model A4, the correlation model is more appropriately reselected, in a case where the analysis result is in a predetermined range, and then the constituent amount of the protein of object S is reanalyzed. Therefore, it is possible to measure the constituent amount of the protein of object S with higher accuracy.

(4) The constituent amount of the protein of object S is analyzed using general-purpose model A4, which is the first correlation model that has high general-purpose property corresponding to all of the samples. Then, general-purpose model A5, which is the second correlation model corresponding to the composition ratio of the protein of object S, is reselected based on the analysis result as necessary, and then the constituent amount of the protein of object S is analyzed. In other words, the constituent amount of the protein of object S is analyzed without changing the correlation model based on the analysis result when general-purpose model A4 is used, or the constituent amount of the protein of object S is analyzed after the correlation model is more appropriately reselected. Therefore, it is possible to maintain reliability of the measurement of the constituent amount of the protein of object S, and it is possible to increase throughput of the analysis process.

(Other Embodiments)

Food analysis apparatus 100 includes an exemplary embodiment other than Exemplary Embodiments 1 to 8 described above. Hereinafter, the exemplary embodiments other than Exemplary Embodiments 1 to 8 described above of food analysis apparatus 100 represent modification example of Exemplary Embodiments 1 to 8 described above.

In Exemplary Embodiment 2 described above, as in the following equation (2), the first dedicated model and the second dedicated model may be generalized and expressed by a polynomial equation in which the absorbance of the sample with respect to light having a plurality of wavelength.

$$Y = K0 + K1 \cdot f1\,(\lambda 1,\,T) + K2 \cdot f2\,(\lambda 2,\,T) \cdots + Kn \cdot fn\,(\lambda n,\,T) \cdots (2)$$

[Y: correlation model, fm $(\lambda m, T)$ (m = 1, 2, ... n): absorbance of sample with respect to light having wavelength $\lambda m$ when a temperature of the sample is T, Km (m = 0, 1, 2, ... n): proportional constant]

In addition, special functions g(T) and h(T) are combined to the polynomial equation in which the absorbance of the sample is used with respect to the light having the plurality of wavelengths and, as in the following Equation (3), the first dedicated model and the second dedicated model are further generalized and expressed.

$$Y = K0 + g\,(T) \cdot K1 \cdot f1\,(\lambda 1) + h(T) \cdot K2 \cdot f2\,(\lambda 2) + \cdots + Kn \cdot fn\,(\lambda n) \cdots (3)$$

[Y: correlation model, fm $(\lambda m)$ (m = 1, 2, $\cdots$ n): absorbance of sample with respect to light having wavelength $\lambda m$, g(T): when T $\geq$ 0°C, "1", and when T < 0°C, "0", h(T): when T $\geq$ 0°C, "0", and when T < 0°C, "1", Km (m = 0, 1, 2, $\cdots$ n): proportional constant]

In Exemplary Embodiment 7 described above, the samples, which define the general-purpose model, are classified for each type of food such as vegetable, meat, or fish, then, the dedicated model may be calculated in advance as the correlation model representing the correlations between the absorbance of the samples and the constituent amount of the samples, with respect to each group of the classified samples.

In this case, for example, when green object S is the analysis object, the constituent amount of object S is analyzed using the dedicated model corresponding to a group of samples of vegetables. In addition, when red object S is the analysis object, the constituent amount of object S is analyzed using the dedicated model corresponding to a group of samples of meat. In addition, when blue object S is the analysis object, the constituent amount of object S is analyzed using the dedicated model corresponding to a group of samples of fish.

In addition, a user may set types of groups, or a wavelength of a certain constituent (for example, dietary fiber from a vegetable), which is a feature of the respective groups other than a nutritional constituent such as protein, may be statistically calculated, and analysis may be performed using the presence or absence of the wavelength and the absorbance.

In general, there is a correlation between a reflection ratio of the sample with respect to the light having the wavelength in the near-infrared region and color density of the sample. Therefore, correlations between the absorbance and the constituent amount of the sample tend to significantly differ from each other, depending on the color density of the sample.

Accordingly, in Exemplary Embodiment 7 described above, the samples, which define the general-purpose model, are classified depending on the color density of the samples, and with respect to the respective groups of the classified samples, the general-purpose model may be calculated in advance as the correlation model representing the correlation between the absorbance of the samples and the constituent amount of the samples.

In this case, when deep-color object S is the analysis object, it is preferable that the constituent amount of object S is analyzed using the dedicated model corresponding to a group of deep-color samples. In addition, when light-color object S is the analysis object, it is preferable that the constituent amount of object S is analyzed using the dedicated model corresponding to a group of light-color samples.

In Exemplary Embodiment 8 described above, a correlation model as an object, to which the absorbance of object S is applied, may be switched depending on whether or not a composition ratio of the lipid and the carbohydrate of object S is equal to or higher than a threshold value.

In the exemplary embodiments described above, the dedicated model corresponding to the feature information of an analysis site of object S may be selected from a plurality of dedicated models stored in storage unit 30. Then, the absorbance of the analysis site of object S, which is calculated based on an amount of light received by light receiving element 22A corresponding to the analysis site of object S, may be applied to the selected dedicated model.

For example, in Exemplary Embodiment 1 described above, as shown in FIG. 6, two fish S1 and S2 are mounted on table 13 as object S, one fish S1 is in the frozen state and the other fish S2 is in the unfrozen state.

In this case, the absorbance of fish S1 in the frozen state is calculated based on an amount of light reception measured by light receiving element 22A of light receiving unit 15. The calculated absorbance of fish S1 is applied to first dedicated model A2, which is the second correlation model, created based on the samples in the frozen state, and thereby the constituent amount of fish S1 is analyzed.

By comparison, the absorbance of fish S2 in the unfrozen state is calculated based on an amount of light reception measured by light receiving element 22A of light receiving unit 15. The calculated absorbance of fish S2 is applied to second dedicated model A3, which is the second correlation model, created based on the samples in the unfrozen state, and thereby the constituent amount of fish S2 is analyzed.

By such configuration, the constituent amount of the analysis site of object S is analyzed using the correlation model more appropriately suitable for the feature information of the analysis sites of object S. Therefore, it is possible to measure the constituent amount of object S with higher accuracy.

In exemplary embodiments described above, the constituent amount of the respective constituents (protein, lipid, and carbohydrate) of object S and the respective calorie coefficients are multiplied and a value of calories of object S, which is a sum of the multiplied values, may be calculated as nutrition information.

In this case, in Exemplary Embodiment 8 described above, analysis unit 31 may be configured to, first, apply the absorbance of object S to general-purpose model A4, and thereby to analyze a value of calorie of object S. Then, when the values of the calories of object S are equal to or higher than the threshold value, analysis unit 31 may be configured to switch the correlation models as objects to which the absorbance of object S is applied.

In addition, when the values of the calories of object S are equal to or less than the threshold value, the analysis unit may be configured to switch between the correlation models as an object to which the absorbance of object S is applied. For example, when the values of the calories of object S are very low, it is highly possible for object S to include a vegetable having low calories. Therefore, in this case, the value of the calorie of object S may be calculated using the dedicated model corresponding to the group of the samples of vegetables.

In exemplary embodiments described above, protein, lipid, and carbohydrate of object S are analyzed; however, instead of or in addition to the constituents above, constituents such as salt, various minerals, and vitamin of object S may be analyzed.

In exemplary embodiments described above, the absorbance of the samples maybe classified in association with the feature information including a plurality of items of information of a state, a temperature, a light intensity, a weight, and a color of the samples. In this manner, with respect to the classified samples, the dedicated model may be calculated in advance as the correlation model representing the correlation between the absorbance of the samples and the constituent amounts of the samples. In this case, the dedicated model as the object, to which the absorbance of object S is applied, is switched depending on the feature information of object S.

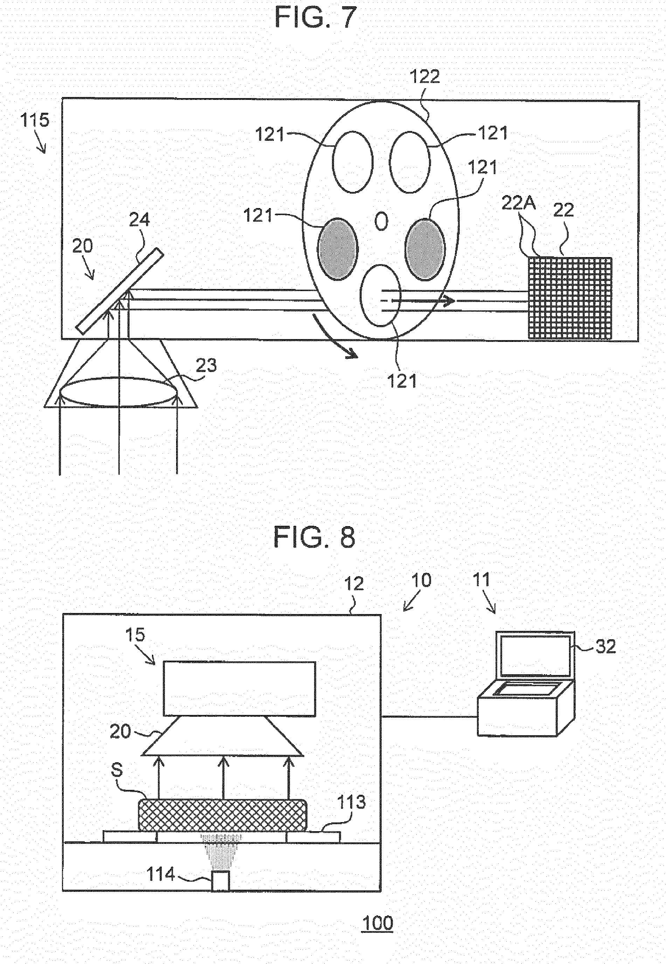

In exemplary embodiments described above, as shown in FIG. 7, light receiving unit 115 may include a plurality of filters 121 that selectively transmit light having a specific wavelength. In this case, light receiving unit 115 may include a filter switching unit 122 that switches between filters 121 disposed on an optical path of light to light receiving sensor 22. Note that, in a case where the filters are disposed, in general, properties are degraded when an incident angle is changed. Therefore, it is preferable that an optical system such as a lens, which adjusts the incident angle with respect to the filter, is provided.

In exemplary embodiments described above, as shown in FIG. 8, food analysis apparatus 100 may have a configuration in which light emitting unit 114 is provided below table 113 and light transmitted through object S is received by light receiving unit 15.

In exemplary embodiments described above, food analysis apparatus 100 may transmit the analysis result of nutrition information of object S to an external device via network. Then, the external device may be configured to manage history of the analysis results.

As described above, food analysis apparatus 100 of the present disclosure has the following configuration and effects. Food analysis apparatus 100 is a food analysis apparatus that analyzes nutrition information of food, and includes an irradiation unit that irradiates the food with light having a wavelength in a near-infrared region; and a light receiving unit that receives light transmitted through the food or light reflected from the food, of light emitted from the irradiation unit. Food analysis apparatus 100 further includes a model storage unit (storage unit 30) which is classified in association with feature information including at least one item of information of state, temperature, light intensity, weight, and color of the sample, a plurality of correlation models including a correlation model representing a correlation between nutrition information of the sample and the classified absorbance of the sample, in which, the absorbance of a sample that is calculated based on an amount of light reception measured by the light receiving unit when the irradiation unit irradiates light to the plurality of samples of which the nutrition information is already known. Food analysis apparatus 100 further includes analysis unit 31 configured to analyze nutrition information of the food by selecting a correlation model corresponding to the nutrition information of the food from the plurality of correlation models stored in the model storage unit, and applying, to the selected correlation model, the absorbance of the food which is calculated based on the amount of light received by the light receiving unit when the food is irradiated with light.

By such configuration, the correlation model corresponding to the feature information of the food as the object for the analysis is selected from the plurality of correlation models stored in the model storage unit. Then, the nutrition information of the food is analyzed using the selected correlation model. Therefore, it is possible to measure the nutrition information of the food as the object for the analysis with higher accuracy, compared to a case where the correlation model is uniformly applied so as to analyze the nutrition information of the food, regardless of the feature information of the food as the object for the analysis.

In food analysis apparatus 100 of the present invention, based on an analysis result of nutrition information of the food obtained when the absorbance of the food, which is calculated based on an amount of light reception measured by the light receiving unit which is obtained when the irradiation unit irradiates the food with light, is applied to a first correlation model, the analysis unit 31 determines whether or not an object, to which the absorbance of the food is applied, is switched from the first correlation model (general-purpose model A1) to second correlation model (first dedicated model or second dedicated model).

By such configuration, the correlation model is appropriately reselected as necessary based on the analysis result of the nutrition information of the food as the object for the analysis, and then the nutrition information of the food is reanalyzed. Therefore, it is possible to measure the nutrition information of the food as the object for analysis with higher accuracy.

In food analysis apparatus 100 of the present invention, after the absorbance of the sample, which is calculated based on an amount of light reception measured by the light receiving unit, which is obtained when the irradiation unit irradiates the plurality of samples with light, is classified based on whether or not the nutrition information of the sample is within a predetermined range, the model storage unit (storage unit 30) stores, as the second correlation model, a correlation model representing a correlation between the nutrition information of the samples and the absorbance of the samples classified to be within the predetermined range. The analysis unit 31 switches the target, to which the absorbance of the food is applied, from the first correlation model to the second correlation model, when the analysis result of the nutrition information of the food is within the predetermined range, which is obtained when the absorbance of the food, which is calculated based on an amount of light received by the light receiving unit when the irradiation unit irradiate the food with light, is applied to the first correlation model.

By such configuration, when the analysis result of the nutrition information of the food as the object for the analysis is within the predetermined range, the nutrition information of the food is analyzed using the correlation model corresponding to the samples of which the nutrition information is within the same range, of the plurality of samples. In other words, when the absorbance of the food as the object for the analysis is applied to the first correlation model, the correlation model is more appropriately reselected in the case where the analysis result is within the predetermined range, and then the nutrition information of the food is reanalyzed. Therefore, it is possible to measure the nutrition information of food as the object for analysis with higher accuracy.

In food analysis apparatus 100, the first correlation model represents a correlation between the entire absorbance of a sample and nutrition information of the sample that defines the plurality of correlation models, and the second correlation model represents a correlation between a part of the absorbance of a sample and nutrition information of the sample that defines the plurality of correlation models.

By such configuration, first, the nutrition information of the food as the object for the analysis is analyzed using the correlation model having high general-purpose properties corresponding to all of the samples. Then, the correlation model corresponding to the samples classified in association with the feature information of the food as the object for the analysis as necessary is reselected, based on the analysis result, and then the nutrition information of the food is reanalyzed. In other words, when the correlation model having high general-purpose properties is used, the nutrition information of the food is analyzed without switching the correlation model based on the analysis result, or the correlation model is more appropriately reselected, and then the nutrition information of the food is reanalyzed. Therefore, it is possible to maintain reliability of the measurement of the nutrition information of the food as the object for the analysis, and it is possible to increase throughput of the analysis process.

In food analysis apparatus 100 of the present disclosure, the light receiving unit includes a plurality of light receiving elements that receive light transmitted through an analysis site of the food and light reflected from the analysis site of the food. In addition, the analysis unit 31 selects a correlation model corresponding to feature information of the analysis site of the food, from the plurality of correlation models stored in the model storage unit, and applies, to the selected correlation model, the absorbance of the analysis site of the food which is calculated based on the amount of light received by the light receiving element corresponding to the analysis site of the food, and thereby analyzes nutrition information of the analysis site of the food.

By such configuration, the nutrition information of the analysis site of the food is analyzed using the correlation model more suitable to the feature information of the analysis sites of the food as the object for the analysis, compared to a case where the same correlation model is uniformly applied to all of the foods as the object for the analysis. Therefore, it is possible to measure the nutrition information of the food as the object for the analysis with higher accuracy.

INDUSTRIAL APPLICABILITY

As described above, according to the present invention, there is provided a food analysis apparatus which achieves special effects including that it is possible to avoid an error in an analysis result occurring in a case where a state of food as an object for analysis significantly differs from a state of a sample used to create a regression equation, and thus, it is possible to measure nutrition information of the food as the object for the analysis with higher accuracy. Hence, information such as calories or a constituent amount of food is widely used for an analysis apparatus or the like that calculates an analysis object in a non-destructive manner.

REFERENCE MARKS IN THE DRAWINGS

| | |
|---|---|
| 10 | measurement unit |
| 11 | analysis unit |
| 12 | housing |
| 13 | table |
| 14 | light emitting unit (irradiation unit) |
| 15 | light receiving unit |
| 20 | light collecting unit |
| 21 | spectroscopic unit |
| 21A | first spectroscopic unit |
| 21B | second spectroscopic unit |
| 21C | third spectroscopic unit |
| 22 | light receiving sensor |
| 22A | light receiving element |
| 23 | collecting lens |
| 24 | reflective unit |
| 30 | storage unit (model storage unit) |
| 31 | analysis unit |
| 32 | display unit |
| 113 | table |
| 114 | light emitting unit (irradiation unit) |
| 115 | light receiving unit |
| 121 | filter |
| 122 | filter switching unit |
| A1 | general-purpose model (first correlation model) |
| A2 | first general-purpose model (second correlation model) |
| A3 | second general-purpose model (second correlation model) |
| A4 | general-purpose model (first correlation model) |
| A5 | dedicated model (second correlation model) |
| C1 to CX | sample (X samples) |
| G1 | group of all of samples C1 to CX |
| G2 | group of frozen samples |
| G3 | group of unfrozen samples |
| L | connection cable |
| S | object |

**Claims**

**1.** A food analysis apparatus (100) configured to analyze nutrition information of food, the apparatus comprising:

an irradiation unit (14) configured to irradiate the food with light containing a wavelength in a near-infrared region;
a light receiving unit (15) configured to receive, among the light emitted from the irradiation unit (14), light transmitted through the food or light reflected from the food;
a model storage unit (30) configured to store a plurality of correlation models representing a correlation between nutrition information and absorbance of the plurality of samples,

wherein the nutrition information includes at least one of protein, lipid and carbohydrate and the absorbance of each of the plurality of samples is:

calculated based on an amount of light reception that is measured by the light receiving unit when the irradiation unit (14) irradiates, with the light, each of the plurality of samples whose nutrition information is already known, and

classified in association with feature information including at least one item of information of a state, a temperature, a light intensity, a weight, and a color of each of the plurality of samples; and

an analysis unit (31) configured to analyze the nutrition information of the food by:

selecting a correlation model from the plurality of correlation models stored in the model storage unit (30), and applying the absorbance of the food which is calculated based on the amount of light received by the light receiving unit (15) when the food is irradiated with the light, to the selected correlation model;

**characterised in that** the analysis unit (31) is further configured to determine whether or not the correlation model to which the absorbance of the food is applied, is switched from a first correlation model to a second correlation model based on an analysis result of the nutrition information of the food obtained when the absorbance of the food is applied to the first correlation model wherein the absorbance of the food is calculated based on the amount of light received by the light receiving unit (15) when the irradiation unit (14) irradiates the food with the light;

wherein the absorbance of each of the plurality of samples calculated based on an amount of light reception that is measured by the light receiving unit (15) when the irradiation unit (14) irradiates, with the light, each of the plurality of samples is classified based on whether or not the nutrition information of corresponding each of the plurality of samples is within a predetermined range;

and **in that** the model storage unit (30) is further configured to store the second correlation model which represents the correlation between the nutrition information and the absorbance of the plurality of samples which is classified as being within the predetermined range;

and **in that** the analysis unit (31) is further configured to switch the correlation model to which the absorbance of the food is applied, from the first correlation model to the second correlation model, in a case where the analysis result of the nutrition information of the food is within the predetermined range when the absorbance of the food is applied to the first correlation model, in which the absorbance of the food is calculated based on the amount of light received by the light receiving unit (15) when the irradiation unit irradiates the food with the light.

2.  The food analysis apparatus (100) according to claim 1, wherein
    the first correlation model represents a correlation between an absorbance of a whole of each of the plurality of samples and the nutrition information of each of the plurality of samples in which the plurality of correlation models are defined by the plurality of samples, and
    wherein the second correlation model represents a correlation between an absorbance of a part of each of the plurality of samples and the nutrition information of the plurality of samples in which the plurality of correlation models are defined by the plurality of samples.

3.  The food analysis apparatus (100) according to claim 1 or 2,
    wherein the light receiving unit (15) comprises a plurality of light receiving elements (22) configured to receive light transmitted through an analysis site of the food and light reflected from the analysis site of the food; and
    wherein the analysis unit (31) is further configured to analyze nutrition information of the analysis site of the food by selecting a correlation model corresponding to feature information of the analysis site of the food, from the plurality of correlation models stored in the model storage unit (30), and applying, to the selected correlation model, an absorbance of the analysis site of the food which is calculated based on an amount of light received by the light receiving element (22) corresponding to the analysis site of the food.

**Patentansprüche**

1.  Vorrichtung (100) zur Analyse von Lebensmitteln, die zum Analysieren von Nährstoff-Informationen eines Lebensmittels eingerichtet ist, wobei die Vorrichtung umfasst:

eine Bestrahlungs-Einheit (14), die so eingerichtet ist, dass sie das Lebensmittel mit Licht bestrahlt, das eine Wellenlänge in einem nahen Infrarotbereich enthält;

eine Lichtempfangs-Einheit (15), die so eingerichtet ist, dass sie von dem Licht, das von der Bestrahlungs-Einheit (14) emittiert wird, Licht, das von dem Lebensmittel durchgelassen, oder Licht empfängt, das von dem Lebensmittel reflektiert wird;

eine Einheit (30) zur Speicherung von Modellen, die so eingerichtet ist, dass sie eine Vielzahl von Korrelations-Modellen speichert, die eine Korrelation zwischen Nährstoff-Informationen und Absorptionsvermögen der Vielzahl von Proben repräsentieren, wobei die Nährstoff-Informationen Proteine, Lipide oder/und Kohlenhydrate einschließen und das Absorptionsvermögen jeder der Vielzahl von Proben

auf Basis eines Maßes des Lichtempfangs jeder der Vielzahl von Proben berechnet, deren Nährstoff-Informationen bereits bekannt sind, das durch die Lichtempfangs-Einheit gemessen wird, wenn durch die Bestrahlungs-Einheit (14) mit dem Licht bestrahlt wird, und

in Verbindung mit Merkmals-Informationen klassifiziert wird, die wenigstens ein Element von Informationen über einen Zustand, eine Temperatur, eine Lichtintensität, ein Gewicht sowie eine Farbe jeder der Vielzahl von Proben einschließen; und

eine Analyse-Einheit (31), die so eingerichtet ist, dass sie die Nährstoff-Informationen des Lebensmittels analysiert, indem sie

ein Korrelations-Modell aus der in der Einheit (30) zur Speicherung von Modellen gespeicherten Vielzahl von Korrelations-Modellen auswählt, und

das Absorptionsvermögen des Lebensmittels, das auf Basis der durch die Lichtempfangs-Einheit (15) empfangenen Lichtmenge berechnet wird, wenn das Lebensmittel mit dem Licht bestrahlt wird, auf das ausgewählte Korrelations-Modell anwendet;

**dadurch gekennzeichnet, dass**

die Analyse-Einheit (31) des Weiteren so eingerichtet ist, dass sie auf Basis eines Analyse-Ergebnisses der Nährstoff-Informationen des Lebensmittels, die ermittelt werden, wenn das Absorptionsvermögen des Lebensmittels auf das erste Korrelations-Modell angewendet wird, feststellt, ob das Korrelations-Modell, auf das das Absorptionsvermögen des Lebensmittels angewendet wird, von einem ersten Korrelations-Modell auf ein zweites Korrelations-Modell umgestellt wird, wobei das Absorptionsvermögen des Lebensmittels auf Basis der durch die Lichtempfangs-Einheit (15) empfangenen Lichtmenge beim Bestrahlen des Lebensmittels mit dem Licht durch die Bestrahlungs-Einheit (14) berechnet wird;

und dadurch, dass anhand des Absorptionsvermögens jeder der Vielzahl von Proben, das auf Basis eines Maßes des Lichtempfangs berechnet wird, das durch die Lichtempfangs-Einheit (15) gemessen wird, wenn die Bestrahlungs-Einheit (14) mit dem Licht bestrahlt, jede der Vielzahl von Proben basierend darauf klassifiziert wird, ob die Nährstoff-Informationen, die jeder der Vielzahl von Proben entsprechen, innerhalb eines vorgegebenen Bereiches liegen;

und dadurch, dass die Einheit (30) zur Speicherung von Modellen des Weiteren so eingerichtet ist, dass sie das zweite Korrelations-Modell speichert, das die Korrelation zwischen den Nährstoff-Informationen und dem Absorptionsvermögen der Vielzahl von Proben repräsentiert, die als innerhalb des vorgegebenen Bereiches liegend klassifiziert werden;

und dadurch, dass die Analyse-Einheit (31) des Weiteren so eingerichtet ist, dass sie das Korrelations-Modell, auf das das Absorptionsvermögen des Lebensmittels angewendet wird, von dem ersten Korrelations-Modell auf das zweite Korrelations-Modell umstellt, wenn das Ergebnis der Analyse der Nährstoff-Informationen des Lebensmittels innerhalb des vorgegebenen Bereiches liegt, wenn das Absorptionsvermögen des Lebensmittels auf das erste Korrelations-Modell angewendet wird, wobei das Absorptionsvermögen des Lebensmittels auf Basis der Menge an Licht berechnet wird, die durch die Lichtempfangs-Einheit (15) empfangen wird, wenn die Bestrahlungs-Einheit das Lebensmittel mit dem Licht bestrahlt.

2.  Vorrichtung (100) zur Analyse von Lebensmitteln nach Anspruch 1, wobei

das erste Korrelations-Modell eine Korrelation zwischen einem Absorptionsvermögen einer Gesamtheit jeder der Vielzahl von Proben und den Nährstoff-Informationen jeder der Vielzahl von Proben repräsentiert und dabei die Vielzahl von Korrelations-Modellen durch die Vielzahl von Proben definiert wird, und

das zweite Korrelations-Modell eine Korrelation zwischen einem Absorptionsvermögen eines Teils jeder der Vielzahl von Proben und den Nährstoff-Informationen der Vielzahl von Proben repräsentiert und dabei die Vielzahl von Korrelations-Modellen durch die Vielzahl von Proben definiert wird.

**3.** Vorrichtung (100) zur Analyse von Lebensmitteln nach Anspruch 1 oder 2,
wobei die Lichtempfangs-Einheit (15) eine Vielzahl von Lichtempfangs-Elementen (22) umfasst, die so eingerichtet sind, dass sie Licht, das durch eine Analyse-Position des Lebensmittels durchgelassen wird, und Licht empfangen, das von der Analyse-Position des Lebensmittels reflektiert wird; und
wobei die Analyse-Einheit (31) des Weiteren so eingerichtet ist, dass sie Nährstoff-Informationen der Analyse-Position des Lebensmittels analysiert, indem sie ein Korrelations-Modell, das Merkmals-Informationen der Analyse-Position entspricht, aus der in der Einheit (30) zur Speicherung von Modellen gespeicherten Vielzahl von Korrelations-Modellen auswählt und auf das ausgewählte Korrelations-Modell ein Absorptionsvermögen der Analyse-Position des Lebensmittels anwendet, das auf Basis der Lichtmenge berechnet wird, die durch das Licht Empfangs-Element (22) empfangen wird, das der Analyse-Position des Lebensmittels entspricht.

**Revendications**

**1.** Appareil d'analyse de nourriture (100) configuré pour analyser de l'information nutritionnelle concernant une nourriture, l'appareil comprenant :

une unité d'irradiation (14) configurée pour irradier la nourriture avec de la lumière contenant une longueur d'onde dans le domaine de l'infrarouge proche ;
une unité de réception de lumière (15) configurée pour recevoir, parmi la lumière émise par l'unité d'irradiation (14), de la lumière transmise à travers la nourriture ou de la lumière réfléchie par la nourriture ;
une unité de stockage de modèles (30) configurée pour stocker une pluralité de modèles de corrélation représentant une corrélation entre l'information nutritionnelle et l'absorbance de la pluralité d'échantillons, dans lequel l'information nutritionnelle comprend au moins une information parmi la teneur en protéines, en lipides et en glucides, et l'absorbance de chaque échantillon de la pluralité d'échantillons est :

calculée sur base de la quantité de réception de lumière qui est mesurée par l'unité de réception de lumière quand l'unité d'irradiation (14) irradie, avec la lumière, chaque échantillon de la pluralité d'échantillons dont l'information nutritionnelle est déjà connue, et
classifiée en association avec de l'information de caractéristique incluant au moins un élément d'information parmi un état, une température, une intensité de lumière, un poids, et une couleur de chaque échantillon de la pluralité d'échantillons ; et

une unité d'analyse (31) configurée pour analyser l'information nutritionnelle de la nourriture par :

sélection d'un modèle de corrélation parmi la pluralité de modèles de corrélation stockés dans l'unité de stockage de modèles (30), et
l'application de l'absorbance de la nourriture, qui est calculée sur base de la quantité de lumière reçue par l'unité de réception de lumière (15) quand la nourriture est irradiée par la lumière, au modèle de corrélation sélectionné ;

caractérisé en que l'unité d'analyse (31) est en outre configurée pour déterminer si le modèle de corrélation auquel l'absorbance de la nourriture est appliquée est commuté ou non d'un premier modèle de corrélation à un deuxième modèle de corrélation sur base d'un résultat d'analyse de l'information nutritionnelle de la nourriture obtenue quand l'absorbance de la nourriture est appliquée au premier modèle de corrélation, dans lequel l'absorbance de la nourriture est calculée sur base de la quantité de lumière reçue par l'unité de réception de lumière (15) quand l'unité d'irradiation (14) irradie la nourriture avec la lumière ;
dans lequel l'absorbance de chaque échantillon de la pluralité d'échantillons calculée sur base d'une quantité de réception de lumière qui est mesurée par l'unité de réception de lumière (15) quand l'unité d'irradiation (14) irradie, avec la lumière, chaque échantillon de la pluralité d'échantillons, est classifiée sur base du fait que l'information nutritionnelle de chaque échantillon de la pluralité d'échantillons correspondant se trouve ou non dans une plage prédéterminée ;
et en ce que l'unité de stockage de modèles (30) est en outre configurée pour stocker le deuxième modèle de corrélation qui représente la corrélation entre l'information nutritionnelle et l'absorbance de la pluralité d'échantillons qui est classifiée comme se trouvant dans la plage prédéterminée ;
et en ce que l'unité d'analyse (31) est en outre configurée pour commuter le modèle de corrélation auquel l'absorbance de la nourriture est appliquée du premier modèle de corrélation au deuxième modèle de corrélation, dans le cas où le résultat d'analyse de l'information nutritionnelle de la nourriture se trouve dans la plage prédéterminée quand l'absorbance de la nourriture est appliquée au premier modèle de corrélation, dans lequel l'absorbance de la nourriture est calculée sur base de la quantité de lumière reçue par l'unité de réception de lumière (15) quand l'unité d'irradiation irradie la nourriture avec la lumière.

2. Appareil d'analyse de nourriture (100) selon la revendication 1, dans lequel
le premier modèle de corrélation représente une corrélation entre une absorbance de l'ensemble de chaque échantillon de la pluralité d'échantillons et l'information nutritionnelle de chaque échantillon de la pluralité d'échantillons, dans lequel les modèles de la pluralité de modèles de corrélation sont définis par la pluralité d'échantillons, et
dans lequel le deuxième modèle de corrélation représente une corrélation entre une absorbance d'une partie de chaque échantillon de la pluralité d'échantillons et l'information nutritionnelle de la pluralité d'échantillons, dans lequel les modèles de la pluralité de modèles de corrélation sont définis par la pluralité d'échantillons.

3. Appareil d'analyse de nourriture (100) selon la revendication 1 ou 2,
dans lequel l'unité de réception de lumière (15) comprend une pluralité d'éléments de réception de lumière (22) configurés pour recevoir la lumière transmise à travers un site d'analyse de la nourriture et la lumière réfléchie par le site d'analyse de la nourriture ; et
dans lequel l'unité d'analyse (31) est en outre configurée pour analyser l'information nutritionnelle du site d'analyse de la nourriture en sélectionnant un modèle de corrélation correspondant à de l'information de caractéristique du site d'analyse de la nourriture, parmi la pluralité de modèles de corrélation stockés dans l'unité de stockage de modèles (30), et pour appliquer au modèle de corrélation sélectionné une absorbance du site d'analyse de la nourriture, qui est calculée sur base d'une quantité de lumière reçue par l'élément de réception de lumière (22) correspondant au site d'analyse de la nourriture.

# FIG. 1

100

# FIG. 2

# FIG. 3

22

LIGHT RECEIVING
SENSOR

11

STORAGE
UNIT

30

ANALYSIS
UNIT

31

32

DISPLAY
UNIT

# FIG. 4

G1

SAMPLES

C1, C2, C3, C4,···, CX

CLASSIFICATION

FROZEN
SAMPLES

G2

C1, C3, ···

UNFROZEN
SAMPLES

G3

C2, C4, ···

A1

GENERAL-PURPOSE
MODEL

A2

FIRST DEDICATED
MODEL

A3

SECOND DEDICATED
MODEL

# FIG. 5

# FIG. 6

# FIG. 7

# FIG. 8

100

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008122412 A
- JP 2011257218 A